# EUROPEAN PATENT APPLICATION

(11) **EP 2 072 513 A1**
(43) Date of publication of application: **24.06.2009**
(21) Application number: 07150076.3
(22) Date of filing: 17.12.2007
(51) Int. Cl.: C07D 413/10, A61K 31/422, A61K 31/454, A61P 31/00

(54) **A cyano piperidinyl-phenil-oxazolidinone and use thereof**

(71) Applicant: Ferrer Internacional, S.A., 08028 Barcelona (ES)
(72) Inventor: Cano, Montserrat, 08691, Monistrol (Barcelona) (ES); Palomer, Albert, 08014, Barcelona (ES); Guglietta, Antonio, 08750, Molins de Rei (Barcelona) (ES)
(74) Representative: Barlocci, Anna

(57) **Abstract**

The invention provides a compound of formula (I), or any of its enantiomers or mixtures thereof, or their pharmaceutically acceptable salts or solvates, for use as a medicament.

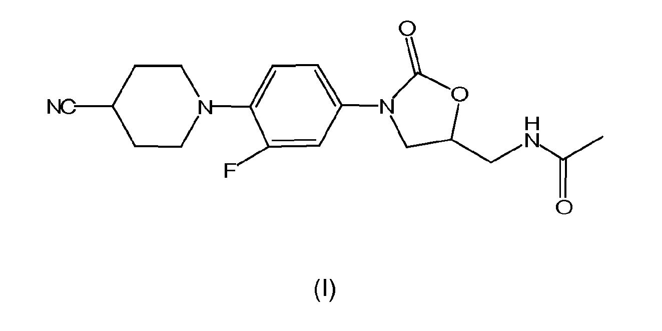

The compound of formula (I) is active against Gram-positive and some Gram-negative human and veterinary pathogens with a weak monoamine oxidase (MAO) inhibitory activity. They are useful for the treatment of bacterial infections. The invention also discloses polymorphic forms of the compound (S)-N-{3-[4-(4-cyano-piperidin-1-yl)-3-fluoro-phenyl]-2-oxo-oxazolidin-5-ylmethyl}-acetamide.

## Description

The present invention relates to a cyano piperidinyl-phenil-oxazolidinone compound for use as a medicament, as well as to new polymorphic forms thereof and processes for their preparation.

### BACKGROUND ART

Oxazolidinones were originally developed as MAO inhibitors for treatment of depression and Parkinson's disease. MAO is one of the primary enzymes responsible for the catabolism of catecholamines. In humans, MAO occurs in two isoforms, MAO-A and MAO-B. MAO-A preferentially deaminates serotonin (5-HT) and norepinephrine; MAO-B preferentially deaminates phenylethylamine, benzylamine, and, in man, dopamine. Normally MAO-A inhibitors, such as moclobemide or tranylcypromine, have been used as antidepressant agents while MAO-B inhibitors, such as selegiline, have been used preferably in the therapy of Parkinson's disease. US Patent 3655687 discloses 5-hydroxymethyl-3-substituted-2-oxazolidinone derivatives with significant antidepressant activity. A compound disclosed in this patent, toloxatone, 5-(hydroxymethyl)-3-(3-methylphenyl)-2-oxazolidinone, is of particular relevance.

Oxazolidinones are also Gram-positive antimicrobial agents. Oxazolidinones bind to the 50S subunit of the prokaryotic ribosome, preventing formation of the initiation complex for protein synthesis. This is a novel mode of action. Other protein synthesis inhibitors either block polypeptide extension or cause misreading of mRNA. Linezolid (N-[[(5S)-3-[3-fluoro-4-(4-morpholinyl)phenyl]-2-oxo-5-oxazolidinyl]methyl]acetamide) is the first approved antimicrobial oxazolidinone for clinical use in the United States and elsewhere.

Linezolid minimal inhibitory concentrations (MICs) vary slightly with the test mode, laboratory, and significance attributed to thin hazes of bacterial survival, but all workers find that the susceptibility distributions are narrow and unimodal with MIC values between 0.5 and 4 µg/mL for streptococci, enterococci and staphylococci. Full activity is retained against Gram-positive cocci resistant to other antibiotics, including methicillin-resistant staphylococci and vancomycin-resistant enterococci. MICs are 2-8 µg/mL for Moxarella, Pasteurella and Bacteroides spp. but other Gram-negative bacteria are resistant as a result of endogenous efflux activity as well as the intake presented by Gram-negative bacteria outer membrane cell. Linezolid is indicated for the treatment of adult patients with the following infections:
Vancomycin-resistant Enterococcus faecium infections, including concurrent bacteremia; nosocomial pneumonia; complicated skin and skin structure infections; community-acquired pneumonia, including concurrent bacteremia; diabetic foot infections; and uncomplicated skin and skin structure infections. Nonetheless, there exist some bacterial strains causing serious respiratory diseases that are resistant to the treatment with Linezolid. Thus, it is still of interest the design and development of new drugs overcoming this drawback.

On the other hand, it is well known in the art that when a drug, and particularly an antibiotic, is administered to a patient, the following adverse effects, among others, can emerge:
a) direct toxicity: cardiac effects; myelosuppression; ototoxicity; renal toxicity (very ill patients may have impaired absorption, altered clearance due to renal dysfunction (increased risk of adverse events, increased antibiotic doses are often required in burn patients));
b) superinfection: the antibiotics can change the normal microbial flora, allowing overgrowth of opportunistic infections;
c) hypersensitivity; and
d) drug interactions.

Unfortunately, there are other adverse effects related to the ingestion of antibacterial oxazolidinones that must be avoided, such as the interactions with other drugs. It is well-known for the skilled man in the art that when a drug (e.g. an oxazolidinone with antibacterial activity) is added to an already therapeutic regimen, the risk of interaction drug-drug is substantially increased. The main consequence of the interaction between two drugs is that the pharmacological effect is modified by the presence of the second drug. The result of this interaction can be harmful for the patient since said interaction can cause toxicity or a decrease in the efficiency of one the drugs ingested by the patient. Because of this, particular attention has been paid to the question of possible adverse interactions with drugs known to be metabolized by monoamine oxidase in patients who are being treated with oxazolidinones. For instance, it has been observed that linezolid (an oxazolidione with antibacterial activity) causes an enhanced response in patients who are taking certain adrenergic agents, including phenylpropanolamine and pseudoephedrine, and it is specifically noted in the leafleft that the doses of these drugs should be reduced in patients receiving linezolid. The package insert for linezolid warns against combining it with tyramine-rich foods and about being aware of a potential interaction with adrenergic and serotonergic agents and also that the side effects of linezolid may be increased by certain drugs including, among others, monoamine oxidase (MAO) inhibitors (such as selegiline, isocarboxazid, pargyline, phenelzine, and tranylcypromine), being recommended that these combinations must be avoided.

It has been proposed the use of oxazolidinones having a weak MAO activity in order to minimize the secondary effects due to the drug-drug interactions. Thus, the PCT application W02006/010756 discloses oxazolidinones of formula: 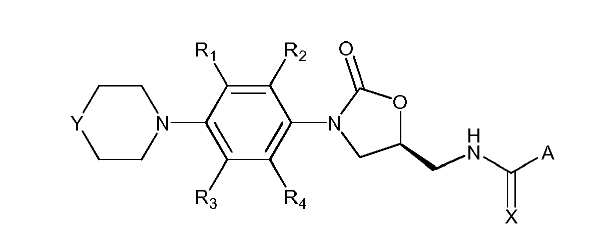 where R₁-R₄, A, X and Y have different values, which are antibacterial agents with a weak MAO activity.

Despite the teaching of the prior art, the research of new antibacterial agents showing a weak inhibitory MAO activity is still an active field.

Moreover, it is also of interest to find pharmaceutically active ingredients that, besides having a determined effect, can be processed and/or administered in solid form, such as tablets or pills, which make pleasant the intake of said active ingredient. Different solid forms of a pharmaceutically active ingredient can have different characteristics, as the skilled man will know, and offer certain advantages, for example with regard to solubility, bioavailability or processing in different galenic formulations. Thus, the discovery of new solid forms allows for improving the characteristics of the pharmaceutical formulations of the active ingredients, since some forms are more adequate for one type of formulation, and other forms for other different formulations. Furthermore, depending on the therapeutic indications, one or another pharmaceutical formulation may be preferred, hence being of great interest the finding of solid forms useful for the manufacture of tablets and pills.

### SUMMARY OF THE INVENTION

Inventors have found that the compound of formula (I) below has a potent antibacterial activity (as it is illustrated below) and a very low MAO inhibitory activity, which implies that potential drug-drug interactions are minimized or eliminated, and therefore, the side effects related to these interactions (pointed out above) are avoided, too. It is of particular relevance the fact that the MAO inhibitory activity of this compound is lower than the MAO inhibitory activity of the compounds of the PCT patent application W02006/010756. Therefore, as it is illustrated below, the compound of the invention shows improved properties over other oxazolidinones with antibacterial activity known in the state of the art.

According to a first aspect of the present invention, it is provided a compound of formula (I), or any of its enantiomers or mixtures thereof, or their pharmaceutically acceptable salts or solvates, for use as a medicament.

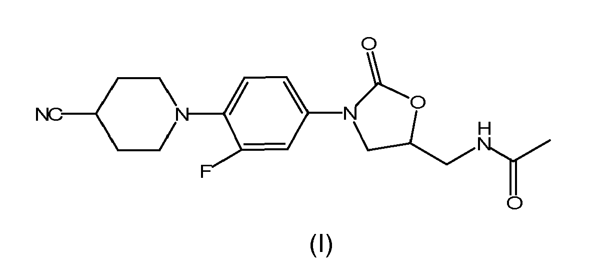

The invention also provides a compound of formula (Ia), which corresponds to the compound (S)-N-{3-[4-(4-cyano-piperidin-1-yl)-3-fluoro-phenyl]-2-oxo-oxazolidin-5-ylmethyl}-acetamide, or its pharmaceutically acceptable salts or solvates.

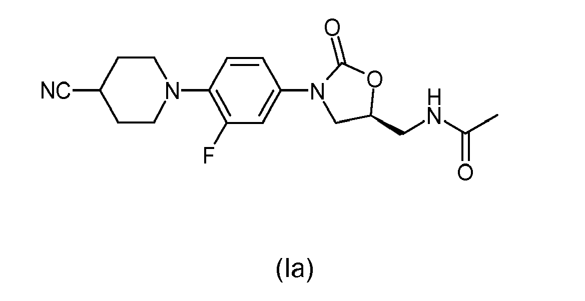

The compound (Ia) is mentioned in the PCT application W02007/023507 as an intermediate of reaction to obtain other oxazolidinones derivatives with antimicrobial activity. However, its therapeutic effects was neither suggested nor disclosed in said prior art. Indeed, in the PCT application W02007/023507 different pathways for the synthesis of oxazolidinones derivatives are taught, starting from a compound such as the one of formula (Ia), but nothing is said about the activity of said starting material and of the possibility of having it in different polymorphic forms.

Inventors have also found that the enantiomer of formula (Ia) of compound (I) can exist in different polymorphic forms. It has been prepared in three stable, well defined and consistently reproducible crystalline forms. It is well known to those skilled in the art in the area of polymorphism that it is not possible to predict the existence of solid forms of a given compound, even if a solid form is already known to exist. Thus, in a second aspect of the invention, it is also provided polymorphic forms of a compound of formula (Ia).

In a third aspect, the invention relates to a polymorphic form B of a compound of formula (Ia), characterized by having an X-ray diffractogram that comprises characteristic peaks at approximately 6.4, 13.4, and 24.6 degrees 2 theta.

In a fourth aspect, the invention relates to a preparation process of the polymorphic form B of the compound of formula (Ia), comprising the following steps: a) mixing a compound of formula (Ia) with a solvent system selected from the group consisting of (C₂-C₄)-alcohols; (C₃-C₆)-ketones and its mixtures with water; (C₂-C₄)-alkanoates of (C₁-C₄)-alkyl and its mixtures with (C₆-C₇)-alkanes; (C₄-C₇)-linear, branched or cyclic ethers or mixtures thereof; b) stirring the mixture from 4 to 14 days in a range of temperature comprised between 17 °C and 30 °C; c) isolating the polymorphic form B of the compound of formula (Ia) obtained in previous step; and d) removing the remaining solvent from the polymorphic form B of the compound of formula (Ia) obtained.

In a fifth aspect, the invention relates to a preparation process of the polymorphic form B of the compound of formula (Ia), comprising the following steps: a) mixing a compound of formula (Ia) with a solvent system selected from the group consisting of a mixture of water/tetrahydrofuran; toluene; and (C₂-C₄)-alkanoates of (C₁-C₄)-alkyl and its mixtures with (C₆-C₇)-alkanes, b) heating to a temperature comprised between 40-70 °C, adding a (C₆-C₇)-alkane in case the (C₂-C₄)-alkanoate of (C₁-C₄)-alkyl is initially used, cooling to a temperature comprised between 0-30 °C and stirring the mixture from 4 to 9 days; c) isolating the polymorphic form B of the compound of formula (Ia) obtained in the previous step; and d) removing the remaining solvent from the polymorphic form B of the compound of formula (Ia) obtained.

In a sixth aspect, the invention relates to a polymorphic form A of a compound of formula (Ia), characterized by having an X-ray diffractogram that comprises characteristic peaks of greater intensity than the rest at approximately 7.1, 16.6, 20.0 and 23.2 degrees 2 theta.

In a seventh aspect, the invention relates to a preparation process of the polymorphic form A of the compound of formula (Ia), comprising the following steps: a) mixing a compound of formula (Ia) with a solvent system selected from the group consisting of water; (C₁-C₄)-alcohol; mixtures of water with (C₁-C₄)-alcohols; and toluene, b) heating to a temperature comprised between 40 °C and 70 °C, and cooling to a temperature comprised between 0-4 °C; c) isolating the polymorphic form A of the compound of formula (Ia) obtained in the previous step; and d) removing the solvent from the polymorphic form A of the compound of formula (Ia) obtained.

In an eighth aspect, the invention relates to a preparation process of the polymorphic form A of the compound of formula (Ia), comprising the following steps: a) mixing a compound of formula (Ia) in a solvent system selected from the group consisting of water, methanol or a mixture of water/tetrahydrofuran, and stirring the mixture at least from 6 to 15 days in a range of temperature comprised between 17 °C and 30 °C; b) isolating the polymorphic form A of the compound of formula (Ia) obtained in previous step; and c) removing the solvent from the polymorphic form A of the compound of formula (Ia) obtained.

In a ninth aspect, the invention relates to a polymorphic form C of a compound of formula (Ia), characterized by having an X-ray diffractogram that comprises characteristic peaks at approximately 8.8, 9.5, 14.4, 20.3 and 23.0 degrees 2 theta.

Another aspect of the present invention relates to a preparation process of the polymorphic form C of the compound of formula (Ia), characterized by comprising the following steps: a) mixing a compound of formula (Ia) in a solvent system selected from the group consisting of (C₂-C₄)-alkanoate of (C₁-C₄)-alkyl , heating up to the boiling point of the solvent system and cooling to a temperature comprised between 0-4 °C; b) isolating the polymorphic form C of the compound of formula (Ia) obtained in the previous step; and c) removing the solvent from the polymorphic form C of the compound of formula (Ia) obtained.

Another aspect of the present invention is a pharmaceutical composition characterized by comprising a therapeutically effective amount of a compound as defined in formula (I) or (Ia) or in any of the polymorphic forms B, A and C, together with appropriate amounts of pharmaceutically acceptable excipients or carriers.

Finally, another aspect of the present invention is the use of a compound selected from: compound of formula (I), compound of formula (Ia), and any of the polymorphs A, B or C of compound of formula (Ia), for the preparation of a medicament for the treatment of bacterial infections of an animal or human. This aspect can also be formulated as compound as defined above for use as bactericide against bacterial infections of an animal or human.

Therefore, the invention is related to a method of treatment and/or prophylaxis of a mammal, including a human, suffering from or being susceptible to bacterial infection, said method comprising the administration to said patient of a therapeutically effective amount of the compounds of the present invention, together with pharmaceutically acceptable diluents or carriers

These and other objects of the present invention will be further described in the detailed description section that follows, and they are not intended to be limiting of the present invention. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the descrip!ion or may be learned by practice of the invention.

### BRIEF DESCRIPTION OF THE DRAWINGS

FIG. 1 shows the X-ray diffraction spectrum of the polymorphic form B, essentially free of solvent system.
FIG. 2 shows the X-ray diffraction spectrum of the polymorphic form A, essentially free of solvent system.
FIG. 3 shows the X-ray diffraction spectrum of the polymorphic form C, essentially free of solvent system.
FIG. 4 is a graphic with the Differential Scanning Calorimetry (DSC) curve of the polymorphic form B. In the same, Tc means corrected temperature in °C, P means "Peak", PH means "Peak Height", A means "Area", ΔH is the melting enthalpy, T in the X-axis shows temperature in °C and HFEU (Y-axis) means "Heat Flow Endo Up" in mW.
FIG. 5 corresponds to the Differential Scanning Calorimetry (DSC) curve of the polymorphic form A. In this Figure, Tc means corrected temperature in °C, P means "Peak", PH means "Peak Height", A means "Area", ΔH is the melting enthalpy, T in the X-axis shows temperature in °C and HFEU (Y-axis) means "Heat Flow Endo Up" in mW.
FIG. 6 shows the Differential Scanning Calorimetry (DSC) curve of the polymorphic form C. In the same, Tc means corrected temperature in °C, P means "Peak", PH means "Peak Height", A means "Area", ΔH is the melting enthalpy, T in the X-axis shows temperature in °C and HFEU (Y-axis) means "Heat Flow Endo Up" in mW.

### DETAILED DESCRIPTION OF THE INVENTION

In a preferred embodiment, the present invention refers to the compound (S)-N-{3-[4-(4-cyano-piperidin-1-yl)-3-fluoro-phenyl]-2-oxo-oxazolidin-5-ylmethyl}-acetamide of formula (Ia).

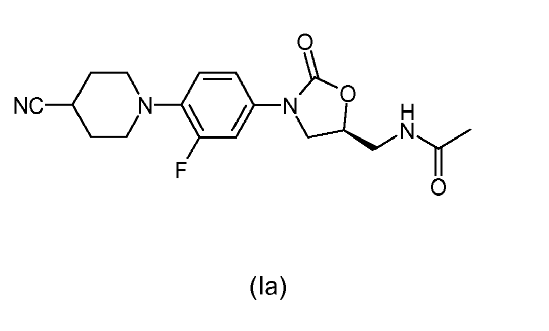

Compound (I) or compound (Ia) can be in form of pharmaceutically acceptable salts or solvates. Examples of pharmaceutically acceptable salts include any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, tartaric acids and the like.

This compound of formula (Ia) exists in different polymorphic forms, namely polymorphic form B, polymorphic form A and polymorphic form C. They have been identified by X-ray diffractogram and by Differential Scanning Calorimetry (DSC).

The data of a X-ray diffractogram for all the polymorphic forms of the compound of formula (Ia) have been obtained using a Bruker D8 Advance diffractometer at a Cu-Kα1 radiation (λ=1.5418Å) and at a power of 35 kV - 45 mA.

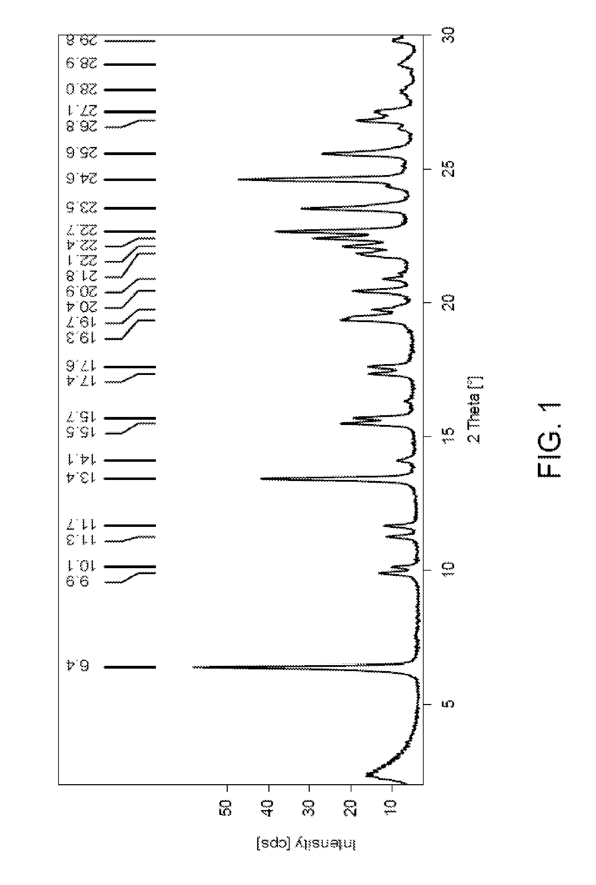

As deduced from FIG. 1, which corresponds to the X-ray diffraction spectrum of polymorphic form B, this form has characteristic peaks found at approximately 6.4, 13.4 and 24.6 degrees 2 theta. As another characteristic feature, other pairs of peaks are observed at 9.9/10.1, 11.3/ 11.7, 15.5/15.7 and 17.4/17.6.

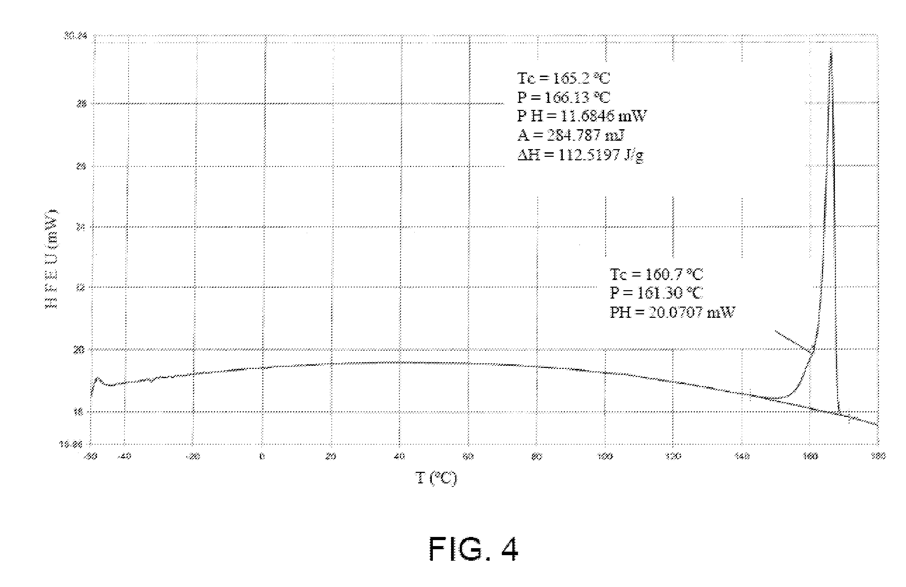

In Differential Scanning Calorimetry (DSC) experiments, as indicated in FIG.4, it is observed that the polymorphic form B has a melting peak at 165-167 °C, which varies slightly with the scanning rate. The melting enthalpy amounts 113-117 J/g.

The data of DSC for all the polymorphic forms of the compound of formula (Ia) have been obtained with a Perkin Elmer DSC 7. The scanning range was from -50 °C to variable end temperatures and the heating rate of 10 °C.min⁻¹ or 80 °C.min⁻¹.

The following Table 1 shows the solubilities of polymorphic form B in different solvent systems, being understood for solvent system any organic or inorganic solvent or a mixture thereof.

**Table 1: Equilibrium solubilities of polymorphic form B.**

| Solvent | Solubility (mg/mL) | pH |
|---|---|---|
| Water (Fluka 95305) | 0.24 | 8.9 |
| Ethanol | 2.6 | -- |
| Ethyl acetate | 2.9 | -- |

Observed in the microscope the polymorphic form B of the compound of formula (Ia) crystallizes as lanceolate needles.

By general way, polymorphic form B is prepared following two different routes and steps starting from a compound of formula (Ia).

As above exposed, polymorphic form B can be obtained by means of suspension equilibration experiments, which means that a solid compound of formula (Ia) is mixed in a proper solvent system and allowed to precipitate under agitation at room temperature (17 °C-30 °C).

To obtain the polymorphic form B, a solid compound of formula (Ia) is mixed in a proper solvent system selected from the group consisting of (C₂-C₄)-alcohols; (C₃-C₆)-ketones and its mixtures with water; (C₂-C₄)-alkanoates of (C₁-C₄)-alkyl and its mixtures with (C₆-C₇)-alkanes; (C₄-C₇)-linear, branched or cyclic ethers or mixtures thereof; and stirring the mixture from 4 to 14 days in a range of temperature comprised between 17 °C and 30 °C. Finally the polymorphic form B of the compound of formula (Ia) obtained is isolated from the medium, for instance by filtration and the remaining solvent in the solid is removed, for instance by drying the compound obtained in a oven.

In a preferred embodiment, polymorphic form B is prepared by means of a process including the steps of:
a) mixing a compound of formula (Ia) in ethanol and stirring the mixture from 5 to 7 days in a temperature range between 17 °C to 30 °C;
b) isolating the polymorphic form B so obtained from ethanol by filtering the mixture, and
c) removing the solvent from the polymorphic form B drying the same under vacuum.

Another way to obtain the polymorphic form B of a compound of formula (Ia) is by re-crystallization processes, in which processes, the compound of formula (Ia) is solubilized by stirring and heating the mixture, allowing the re-crystallization by downloading the temperature. Thus, the preparation process includes the first step of mixing a compound of formula (Ia) with a solvent system selected from the group consisting of a mixture of water/tetrahydrofuran; toluene; and (C₂-C₄)-alkanoates of (C₁-C₄)-alkyl and its mixtures with (C₆-C₇)-alkanes, heating to a temperature comprised between 40-70° C, adding a (C₆-C₇)-alkane in case of a (C₂-C₄)-alkanoate of (C₁-C₄)-alkyl is initially used, cooling to a temperature comprised between 0-30 °C and stirring the mixture from 4 to 9 days, to finally obtain the desired solid form, which in turn is isolated from the medium and the remaining solvent in the solid is removed as in the previous process.

In one particular embodiment, polymorphic form B is prepared by means of a process including the steps of: a) mixing a compound of formula (Ia) in a solvent system consisting in a mixture of ethyl acetate/heptane in a 1:1 ratio; b) heating to a temperature comprised between 40-70 °C; c) cooling to a temperature comprised between 17 °C and 30 °C while stirring from 4 to 9 days; d) isolating the polymorphic form B from the medium; and e) removing the remaining solvent, for instance by drying the compound obtained in a oven

Another particular embodiment consists in the preparation of polymorphic form B by: a) mixing a compound of formula (Ia) in a solvent system consisting in ethyl acetate; b) heating to a temperature comprised between 40-70 °C; c) adding heptane to have a ratio ethyl acetate/heptane of 4:1; d) cooling to a temperature comprised between 0-30 °C; e) stirring the mixture from 4 to 9 days, to finally obtain the desired solid form; f) isolating the obtained polymorphic form B from the medium; and g) removing the remaining solvent, for instance by drying the compound in a oven.

In the context of the present invention, ethanol and 2-propanol are preferably used, as (C₂-C₄)-alcohols.

The (C₃-C₆)-ketones include, among others, acetone, diethyl ketone, methyl isobutyl ketone (MIBK) and methyl ethyl ketone (MEK).

Preferred (C₂-C₄)-alkanoates of (C₁-C₄)-alkyl are ethyl acetate, butyl acetate, such as 1-butyl acetate or t-butyl acetate. When these (C₂-C₄)-alkanoates of (C₁-C₄)-alkyl are mixed with (C₆-C₇)-alkanes, heptane is preferred.

Examples of ethers used in the present invention are dioxane, t-butyl methyl ether and diethyl ether.

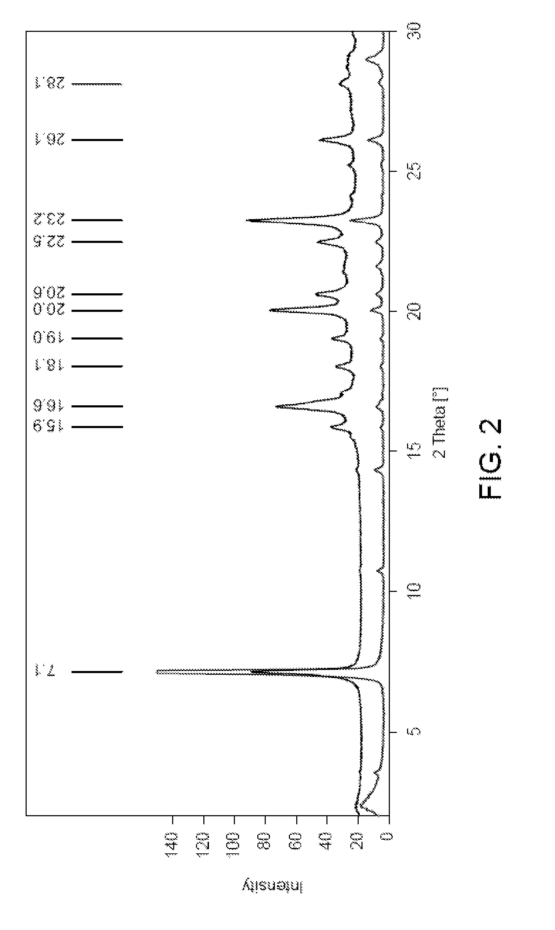

Polymorphic form A, presents an X-ray diffractogram as illustrated in FIG. 2, having characteristic peaks of greater intensity than the rest at approximately 7.1, 16.6, 20.0 and 23.2 degrees 2 theta; and other peaks at approximately 15.9, 18.1, 19,o. 20.6, 22.5 and 28.1 degrees 2 theta.

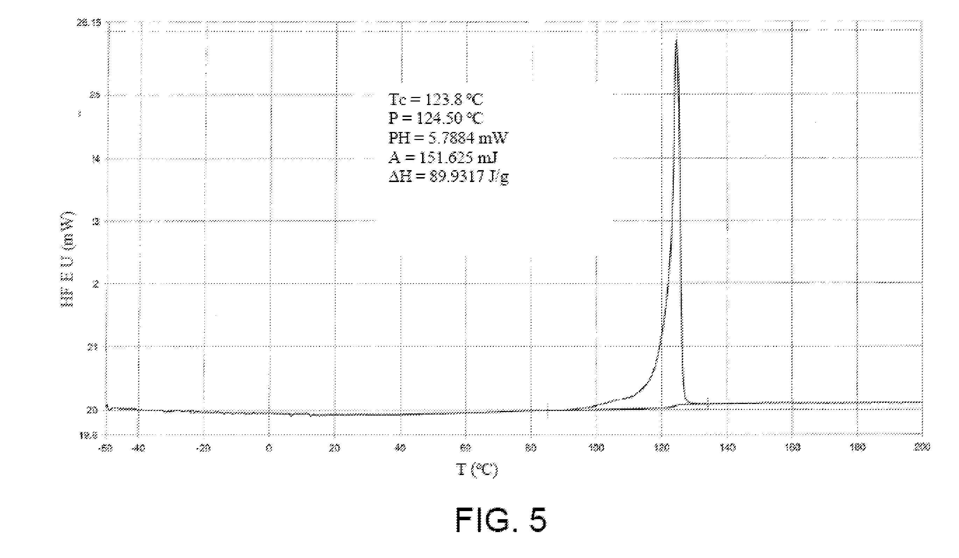

Polymorphic form A, as deduced from FIG. 5, presents a DSC value of 123-124 °C. The total melting enthalpy amounts to 86 to 90 J/g.

Solubility studies were also performed for polymorphic form A. In Table 2 appear the data obtained.

**Table 2: Equilibrium solubilities of polymorphic form A.**

| Solvent | Solubility (mg/mL) | pH |
|---|---|---|
| Water (Fluka 95305) | 0.40 | 8.7 |
| Water (Fluka 95305) | 0.39 | 8.9 |
| Ethanol | 6.3 - 7.3 | -- |
| Ethyl acetate | 11.8 - 15.4 | -- |

By means of microscope observation, the polymorphic form A of the compound of formula (Ia) crystallizes as aggregates of fine particles of poorly defined shape.

In order to obtain said polymorphic form A of compound of formula (Ia), also two ways can be followed.

The first process includes the step of mixing a compound of formula (Ia) with a solvent system selected from the group consisting of water; (C₁-C₄)-alcohol; mixtures of water with (C₁-C₄)-alcohols; and toluene, heating to a temperature comprised between 40 °C and 70 °C and cooling to a temperature comprised between 0-4 °C. Further steps are directed to the isolation of the compound from the medium, for instance by filtration and the removal of the remaining solvent, for instance by drying the compound obtained in a oven.

Preferred solvent systems include ethanol and mixtures of ethanol/water.

Alternatively, the polymorphic form A can be obtained by mixing a compound of formula (Ia) in a solvent system selected from the group consisting of water, methanol or a mixture of water/tetrahydrofuran, and stirring the mixture at least from 6 to 15 days in a range of temperature comprised between 17 °C and 30 °C. Further steps are directed to the isolation of the polymorphic form A from the selected solvent system and the removal of the solvent as in the previous process.

In a particular embodiment, the mixture of a compound of formula (Ia) in a solvent system selected from the group consisting of water, methanol or a mixture of water/tetrahydrofuran is stirred while heated to a temperature between 40 °C and 50 °C allowing further stirring from 6 to 15 days in a range of temperature comprised between 17 °C and 30 °C. Additional steps are directed to the isolation of the polymorphic form A from the selected solvent system and the removal of the solvent as in the previous process.

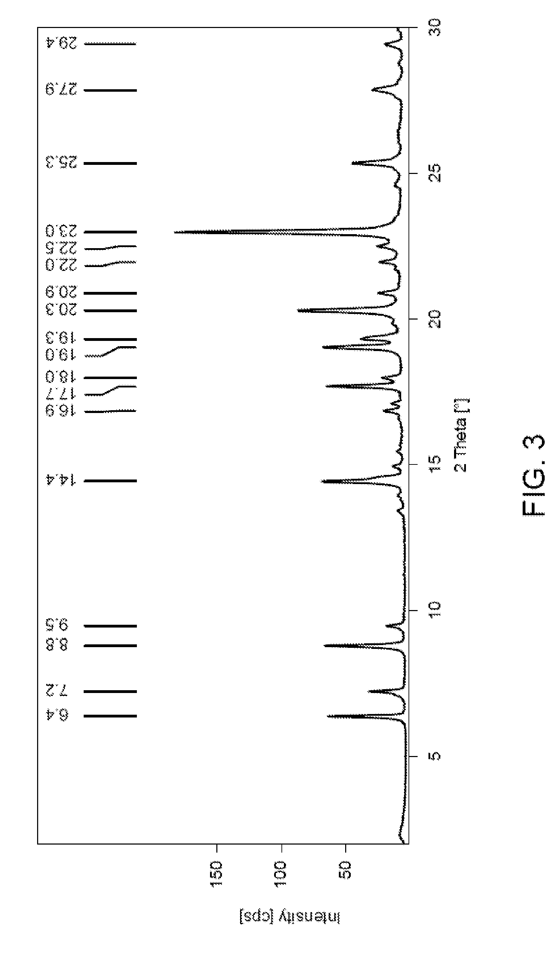

Finally, a new polymorphic form C of the compound of formula (Ia) has been identified. The polymorphic form C is characterized by having a X-ray diffractogram as shown in FIG. 3. The characteristic peaks appear at approximately 8.8, 9.5, 14.4, 20.3 and 23.0 degrees 2 theta. Other significant peaks appear at 17.7-18.0, 19.0-19.3, 25.3 and 27.9 degrees 2 theta.

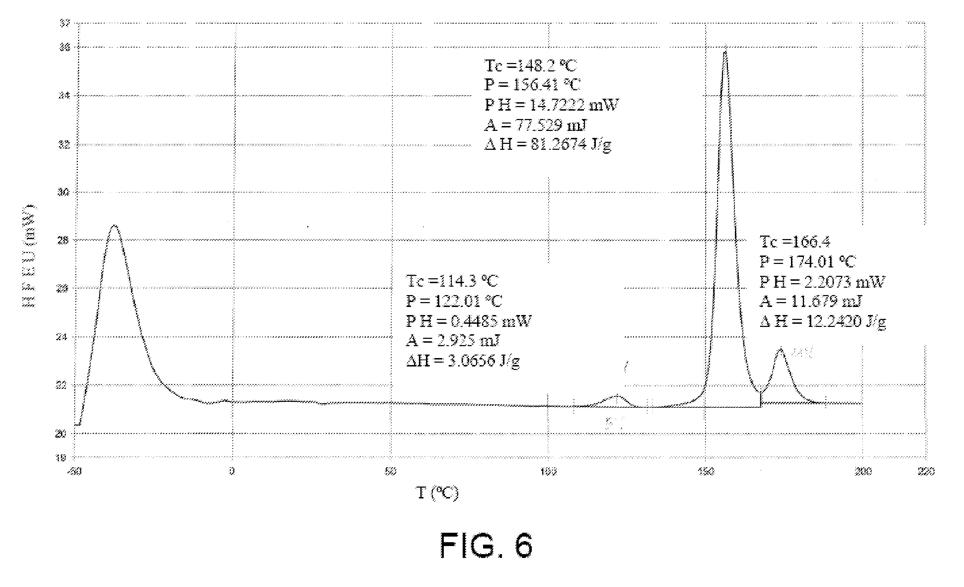

The polymorphic form C presents a Differential Scanning Calorimetry (DSC) that comprises a melting peak at 148 °C with a maximum melting enthalpy of 98 J/g, as shown in FIG. 6.

Viewed in the microscope the polymorphic form C of the compound of formula (Ia) crystallizes as small needles.

Polymorphic form C is preferably obtained in re-crystallization experiments which include the steps of mixing a compound of formula (Ia) in a solvent system selected from the group consisting of 1-butyl acetate, tert-butyl acetate, ethyl acetate and isopropyl acetate , heating up to the boiling point of the solvent system and cooling to a temperature comprised between 0-4 °C. As will be understood by the skilled man in the art, the polymorphic form C obtained is isolated from the medium, preferably by filtration, and the removing solvent of the solid remove preferably by drying.

Suspension equilibrations experiments of polymorphic form A samples in various organic solvents resulted in the formation of polymorphic B. In water, the transformation of pure polymorphic form A into polymorphic form B was apparently hindered kinetically. However, a mixture of polymorphic form A and B in water slowly converted into pure polymorphic form B as well. Similarly, a suspension containing a mixture of polymorphic forms B and C in ethanol converted into polymorphic form B after some time. These experiments indicate that at room temperature (17-30 °C) polymorphic form B is the most stable one of all polymorphic forms of the compound (Ia).

The result from suspension equilibration experiment is additionally confirmed by DSC data. With increasing melting temperature, also the melting enthalpy of the polymorphic form increases. This is characteristic for a monotropic system, which means that the polymorphic form with the highest melting point (form B) is the most stable form at any temperature. Some uncertainty in this interpretation is due to the fact that polymorphic form A was never obtained in completely dry form. The observed melting enthalpy and the true melting point are influenced to some extent by the presence of the hydrate, which is a chemically different entity and therefore not comparable in its thermal properties. However, it seems unlikely that this inaccuracy inverts the order of stability, because the difference in melting temperature and melting enthalpy between polymorphic forms A and B is quite large.

One more confirmation of the relative stability of polymorphic forms A and B is gained from solubility data. Polymorphic form A showed the higher solubility in water, ethanol, and ethyl acetate, which means that it is the less stable form in all of these solvents. The ratio of the solubility values varies from solvent to solvent, which is likely to be due to the fact that the water activity changes from solvent to solvent and that the polymorphic form A is therefore hydrated to different extents. In summary, the stability increases in the order: polymorphic form A < polymorphic form C < polymorphic form B. In a solid state the three polymorphic forms of the present invention have shown to be stable enough to be used and processed in a pharmaceutical formulation.

As above exposed, all the experiments and data shown herewith, indicate that the polymorphic form B of a compound of formula (Ia) is a very stable form. This fact results advantageous because by means of this polymorphic form it is provided a solid form of the compound (S)-N-{3-[4-(4-cyano-piperidin-1-yl)-3-fluoro-phenyl]-2-oxo-oxazolidin-5-ylmethyl}-acetamide that allow for good manipulation for the preparation of solid pharmaceutical formulations.

On the other hand, polymorphic form A of the compound of formula (Ia) shows high solubility in water, ethanol, and ethyl acetate, which can be useful for the formulation of liquid pharmaceutical preparations. At the same time, the high solubility promotes a better in vivo pharmacokinetic profile, which in turn can be derived to an improved activity. Another advantage of polymorphic form A lies in the fact that it is obtained by means of processes in which water and or alcoholic solvents are used, thus making it friendly and pleasant to manufacture.

Polymorphic form C represents also an easy to manipulate solid and stable form of compound of formula (Ia), hence it can also be useful in pharmaceutical compositions.

Moreover, the three polymorphic forms of the present invention have another additional advantage, given that its preparation process is reproducible and robust, and, therefore, easily industrializable.

The pharmaceutical composition of the invention comprises a therapeutically effective amount of a compound formula (I) or a compound of formula (Ia) as defined above or in any of the polymorphic forms B, A and C, together with appropriate amounts of pharmaceutically acceptable excipients or carriers.
The compounds of the present invention can be normally formulated in accordance with standard pharmaceutical practice.

The compounds and compositions of the present invention are useful in the treatment of conditions such as nosocomial pneumoniae, community acquired pneumoniae, caused by methicillin-resistant Staphylococcus aureus (MRSA), including concurrent bacteremia, penicillin resistance and sensitive streptococcus pneumoniae, diabetic foot infections and skin structure infections, and all other infections caused by bacteria sensitive to the compounds described in the invention. The compounds of the present invention are effective against a number of human or animal pathogens, clinical isolates, including vancomycin-resistant organisms and methicillin-resistant organisms. The pharmacological activity of the compounds of the present invention has been determined as shown in the Examples 10 and 11.

Of particular relevance is the activity of the compounds and compositions of the present invention in infections and diseases caused by Staphylococcus. aureus clinical strain cfr methylase, Bacteroides fragillis, Moraxella catarrhalis, Streptococcus pneumoniae and Enterococcus faecium.

Moreover, the compounds and compositions of the present invention show a very high activity (as indicated below) against diseases and infections caused by Staphylococcus. aureus clinical strain cfr methylase, a strain that on the other hand is highly resistant to the treatment with Linezolid.

### EXAMPLES

The following are examples of preparation of the different polymorphic forms. The same are provided for illustrative means, and are not meant to be limiting of the present invention.

### Example 1: Preparation process of N-{3-[4-(4-cyano-piperidin-1-yl)-3-fluorophenyl]-2-oxo-oxazolidin-5-ylmethyl}-acetamide

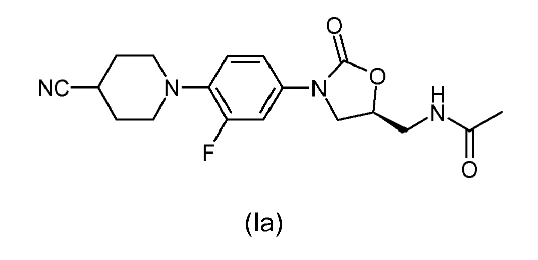

0.098 g (0.28 mmol) of [4-(4-cyano-piperidin-1-yl)-3-fluoro-phenyl]-carbamic acid benzyl ester were dissolved in 0.2 ml of *N*,*N*-dimethyl formamide. To this solution 0.83 ml of a solution of *t*-BuOLi 1 M in Methanol were added. After 30 minutes at room temperature, 0.023 ml of Methanol and a solution of 0.132 g (0.56 mmol) of (S)-N-(3-bromo-2-acetoxipropil)-acetamide were added. The resultant solution was stirred at room temperature for 48 hours. The reaction mixture was transferred to a separator funnel and diluted with 10 ml of dichloromethane. This solution was washed with 3x10 ml of water, dried over MgSO₄, filtered and concentrated under reduced pressure. The resultant material was chromatographied on silica gel eluting with dichloromethane/methanol to give 59 mg (yield= 59%) of N-{3-[4-(4-cyano-piperidin-1-yl)-3-fluoro-phenyl]-2-oxo-oxazolidin-5-ylmethyl}-acetamide.

### Example 2. Preparation process of N-{3-[4-(4-cyano-piperidin-1-yl)-3-fluorophenyl]-2-oxo-oxazolidin-5-ylmethyl}-acetamide.

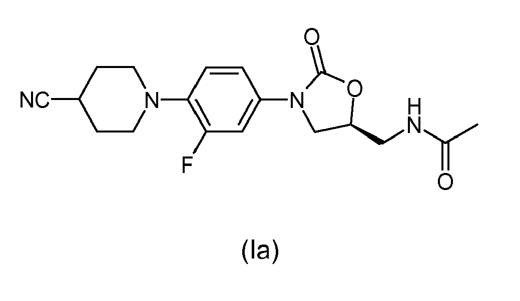

0.200 g (0.63 mmol) of 1-[4-(5-aminomethyl-2-oxo-oxazolidin-3-yl)-2-fluorophenyl]-piperidine-4-carbonitrile were dissolved in 20 ml of ethyl acetate, 0.4 ml of pyridine and 0.24 ml (2.51 mmol) of acetic anhydride were added. After 8 hours the mixture was filtered and the filtrate concentrated in vacuum to give a brown solid. It was purified by chromatography on a silica gel column eluting with a gradient of methanol/dichloromethane. The combined fractions gave a white solid, 133 mg (yield= 59%) of N-{3-[4-(4-cyano-piperidin-1-yl)-3-fluoro-phenyl]-2-oxo-oxazolidin-5-ylmethyl}-acetamide. ¹H NMR (400 MHz, DMSO): δ 1.81 (3H, s), 1.81-1.86 (2H, m), 1.97-1.99(2H, m), 2.88-2.91 (2H, m), 3.05-3.08 (3H, m), 3.37 (2H, t, J= 5.6 Hz), 3.66-3.70 (1 H, m), 4.06 (1 H, t, J= 9.2 Hz), 4.68-4.69 (1 H, m), 7.08 (1 H, t, J= 8.8 Hz), 7.44-7.48 (1 H, m), 7.44-7.48 (1 H, dd, J= 2.4 and 14.8 Hz), 8.29 (1 H, m).
MS (ES) m/z = 361 (MH+). HPLC = 91%

### Example 3: Preparation of a new polymorphic form (form B) of a compound of formula (Ia).

41.9 mg of a solid form of the compound of formula (Ia) with the features of polymorphic form A were added to a solution of ethanol from solubility determination. The mixture was stirred for 6 days in total. The solid obtained was filtered off after 6 days and dried under vacuum for 15 min. The X-ray diffraction technique showed a diffractogram as illustrated in FIG. 1 confirming that the new polymorphic form B of the compound of formula (Ia) had been obtained.

### Example 4: Preparation of a new polymorphic form (form B) of a compound of formula (Ia).

38.2 mg of polymorphic form A of the compound of formula (Ia) were dissolved in 4 mL ethyl acetate at 60 °C. Afterwards, 1 mL heptane was added, leading to slight turbidity, and the solution was rapidly cooled with ice, which led to a gel-like precipitate. The sample was stored at 4 °C for 1 week, leading to the transformation of the gel into a white solid. The white solid was isolated from the solvent system and sepatared from the remaining solvent and shown a diffractogram as illustrated in FIG. 1.

### Example 5: Preparation of a new polymorphic form (form B) of a compound of formula (Ia).

A suspension of 40.8 mg of a compound of formula (Ia) corresponding to polymorphic form A in 1 mL dioxane/tBME 1:4 was stirred at room temperature for 11 days. The solid thus obtained was filtered off and dried. Polymorphic form B of the compound of formula (Ia) was determined under X-ray diffraction.

### Example 6: Preparation of a new polymorphic form (form A) of a compound of formula (Ia).

41.3 mg of a compound of formula (Ia) corresponding to polymorphic form A were dissolved in. 5.2 ml ethanol at 60 °C. The slightly turbid solution was filtered and rapidly cooled with ice. A precipitate formed after short time and was filtered off. By X-ray diffraction the spectra of FIG. 2 was obtained.

### Example 7: Preparation of a new polymorphic form (form A) of a compound of formula (Ia).

40.7 mg of a compound of formula (Ia) corresponding to polymorphic form A were dissolved in 2.2 ml of a mixture ethanol/water 1:1 at 60 °C. The slightly turbid solution was filtered and rapidly cooled with ice. A precipitate formed after short time and was filtered off and analyzed by X.ray diffraction. A diffractogram as that of FIG. 2 was obtained.

### Example 8: Preparation of a new polymorphic form (form A) of a compound of formula (Ia).

43.9 mg of a compound of formula (Ia) were added to the methanol solution from solubility determination, and the mixture was stirred for 12 days in total. The solid was analyzed by Raman spectroscopy after 4 and 12 days and by TG-FTIR after filtration and short drying under vacuum. The X-ray diffraction technique shown a spectra as that of FIG. 2, thus coinciding with the polymorphic form A.

### Example 9. Preparation of a new polymorphic form (form C) of a compound of formula (Ia).

52.4 mg of a compound of formula (Ia) corresponding to the polymorphic form A were dissolved in 5 mL of 1-Butyl acetate at reflux. The solution was allowed to slowly cool down to r.t. (between 17-30 °C) with agitation. A small amount of precipitate formed after 1.5 hours. More white solid formed upon storage at 4 °C for 4 days. The solid was filtered off and dried under vacuum for 4 h. The X-ray diffraction determination gave the spectra of FIG. 3, corresponding to the polymorphic form C of the compound of formula (Ia).

### Example 10: Antibacterial activity

Minimal Inhibitory Concentration (MIC) was determined by using a standard micro dilution method according to The National Committee for Clinical Laboratory Standards (NCCLS), 5^{th} Approved standard M7-A5, 2001, Wayne, PA, USA. Compound (Ia) was tested against Gram-positive and Gram-negative bacteria showing relevant different susceptibility and resistance specifications. The used microorganisms were selected from laboratory reference bacteria and from clinical isolates. One of these strains was a clinical strain of Staphylococcus aureus, linezolid-resistant due to the presence of Cfr, a methyltransferase (Staphylococcus. aureus clinical strain cfr methylase).The tested concentrations were double dilutions from 0.06 µg/mL to 128 µg/mL in 96-well micro titter plates.

The microorganisms used in the study were:
Aerobic Gram-positive bacteria, consisting of Staphylococcus aureus, Staphylococcus epidermidis, Enterococcus faecalis, Enterococcus faecium and Streptococcus pneumoniae and Moraxella catarrhalis, a Gram-negative bacterium, which is relevant to respiratory infections and which is also called fastidious because of its growing requirements. As a representative of anaerobic bacteria Bacteroides fragilis was chosen
MICs were determined in the Brucella Blood medium supplemented for the anaerobic strains, and in the Mueller-Hinton culture medium (cation-adjusted) for the aerobic bacteria.

The tested compound was dissolved in DMSO, and was diluted as far as 2560 µg/ml with the different media according to the specific requirements for each group of strains. The 96-well sealed micro titter plates containing bacteria were incubated in different laboratory conditions depending on the nature of the micro organism. Thus, the aerobic bacteria were incubated during 16-24 h at 35 °C and the so-called fastidious bacteria, such as M. catarrhalis and S. pneumoniae, during 20-24h at 35°C in a microaerobiotic atmosphere containing 5% CO₂ (Anaerocult C, MERCK).

The results of this test are given in Table 3.

**Table 3. In vitro activities against bacteria, MIC (µg/mL)**

| ***In vitro* activities against bacteria, MIC (µg/mL)** | | |
|---|---|---|
| *Microorganism* | Compound (Ia) | Linezolid |
| S. aureus ATCC 25923 MS 319 | 1 | 1 |
| S. aureus ATCC 43300 MR 214 | 1 | 1 |
| S. aureus clinical strain cfr methylase 432 | 2 | 16-32 |
| S. epidermidis ATCC 12228 MR 11 | 1 | 1 |
| S. pneumoniae ATCC 49619 PR 215 | 0.5 | 1 |
| E. faecalis ATCC 29212 53 | 1 | 0.50 |
| E. faecium ATCC 10541 13 | 0.5 | 1 |
| B. fragilis ATCC 25285 A12 | 1 | 2 |
| Moraxella catarrhalis HCl-78 259/339 | 1 | 2 |

As can be derived from Table 3, the compound of formula (Ia) shows a high activity against several bacterial strains and particularly has a specially good activity (MIC of 2 µg/mL) against a strain of Staphylococcus aureus, named S. aureus clinical strain cfr methylase 432. The compound of formula (Ia) is highly effective for diseases and infections caused by said strain, a strain for which Linezolid is low effective (MIC of 16-32 µg/mL), being S. aureus clinical strain cfr methylase 432 catalogued as resistant to Linezolid.

### Example 11: In Vitro MAO-A and MAO-B enzymatic activity

MAO-A and MAO-B enzymatic activities were measured using membranes obtained from SF9 cells expressing either human MAO-A or human MAO-B (Gentest, BD, USA). Assays were done in blank 96-well micro titter plates using kynuramine as substrate and measuring the formation of 4-hydroxyquinoline by fluorescence at 340 nm/465 nm. Briefly, membranes with MAO-A (0.006 mg/mL protein) and MAO-B (0.015 mg/mL protein) were incubated with kynuramine, 30 µM, at 37° for 40 min in the presence of the compound in a final volume of 200 µL. Reactions were stopped by adding NaOH 2N and the reaction product, 4-hydroxyquinoline, was determined by fluorometry using a Tecan Ultra reader.

A low Kᵢ value indicates that the tested inhibitor possesses a tight binding ability to MAO enzyme, thus, it is a strong MAO inhibitor.

MAO-A and MAO-B enzymatic activities are shown in Table 4.

**Table 4. Inhibitory activity of human MAO.**

| Compound | MAO-A Ki µM | MAO-B Ki µM |
|---|---|---|
| Example 1 Compound (Ia) | 1.050 | 2.450 |
| Linezolid | 6.960 | 0.480 |

## Claims

1. A compound of formula (I), or any of its enantiomers or mixtures thereof, or their pharmaceutically acceptable salts or solvates, for use as a medicament.

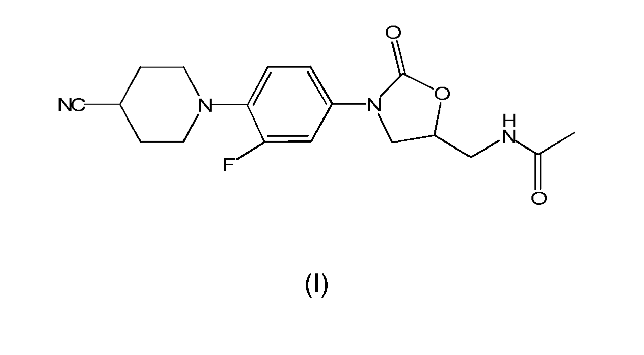

2. The compound according to claim 2, which corresponds to the compound (S)-N-{3-[4-(4-cyano-piperidin-1-yl)-3-fluoro-phenyl]-2-oxo-oxazolidin-5-ylmethyl}-acetamide of formula (Ia).

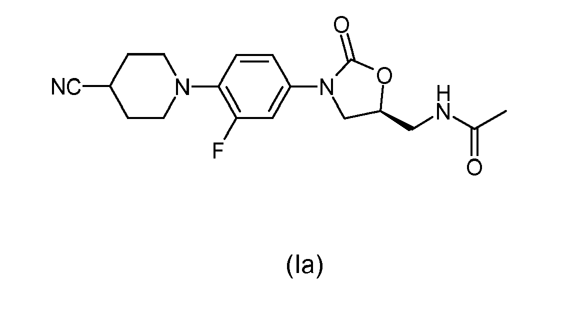

3. A polymorphic form of a compound of formula (Ia).

4. A polymorphic form B of a compound according to claim 3, **characterized by** having an X-ray diffractogram that comprises characteristic peaks at approximately 6.4, 13.4, and 24.6 degrees 2 theta.

5. The polymorphic form according to claim 4, further **characterized by** an X-ray diffractogram as in FIG.1.

6. The polymorphic form according to any of the claims 4-5, further **characterized by** a Differential Scanning Calorimetry (DSC) that comprises a single melting peak at 165-167° C.

7. A preparation process of the polymorphic form B of the compound of formula (Ia) as defined in any of the claims 4-6, comprising the following steps:
a) mixing a compound of formula (Ia) with a solvent system selected from the group consisting of (C₂-C₄)-alcohols; (C₃-C₆)-ketones and its mixtures with water; (C₂-C₄)-alkanoates of (C₁-C₄)-alkyl and its mixtures with (C₆-C₇)-alkanes; (C₄-C₇)-linear, branched or cyclic ethers and mixtures thereof;
b) stirring the mixture from 4 to 14 days in a range of temperature comprised between 17 °C and 30 °C;
c) isolating the polymorphic form B of the compound of formula (Ia) obtained in previous step; and
d) removing the remaining solvent from the polymorphic form B of the compound of formula (Ia) obtained.

8. The process according to claim 7, wherein the step a) is carried out with ethanol and by stirring the mixture from 5 to 7 days in a temperature range between 17 °C to 30 °C.

9. A preparation process of the polymorphic form B of the compound of formula (Ia) as defined in any of the claims 4-6, comprising the following steps:
a) mixing a compound of formula (Ia) with a solvent system selected from the group consisting of a mixture of water/tetrahydrofuran; toluene; and (C₂-C₄)-alkanoates of (C₁-C₄)-alkyl and its mixtures with (C₆-C₇)-alkanes,
b) heating to a temperature comprised between 40-70 °C, adding a (C₆-C₇)-alkane in case the (C₂-C₄)-alkanoate of (C₁-C₄)-alkyl is initially used;
c) cooling to a temperature comprised between 0-30 °C and stirring the mixture from 4 to 9 days;
d) isolating the polymorphic form B of the compound of formula (Ia) obtained in the previous step; and
e) removing the solvent from the polymorphic form B of the compound of formula (Ia) obtained.

10. A polymorphic form A of a compound according to claim 3, **characterized by** having an X-ray diffractogram that comprises characteristic peaks of greater intensity than the rest at approximately 7.1, 16.6, 20.0 and 23.2 degrees 2 theta.

11. The polymorphic form according to claim 10, further **characterized by** an X-ray diffractogram as in FIG.2.

12. The polymorphic form according to any of the claims 10-11, further **characterized by** a Differential Scanning Calorimetry (DSC) that comprises a single melting peak at 123-124 °C.

13. A preparation process of the polymorphic form A of the compound of formula (Ia) as defined in any of the claims 10-12, comprising the following steps:
a) mixing a compound of formula (Ia) with a solvent system selected from the group consisting of water; (C₁-C₄)-alcohols; mixtures of water with (C₁-C₄)-alcohols; and toluene,
b) heating to a temperature comprised between 40 °C and 70 °C, and cooling to a temperature comprised between 0-4 °C;
c) isolating the polymorphic form A of the compound of formula (Ia) obtained in the previous step; and
d) removing the solvent from the polymorphic form A of the compound of formula (Ia) obtained.

14. A preparation process of the polymorphic form A of the compound of formula (Ia) as defined in any of the claims 10-12, comprising the following steps:
a) mixing a compound of formula (Ia) in a solvent system selected from the group consisting of water, methanol or a mixture of water/tetrahydrofuran, and stirring the mixture at least from 6 to 15 days in a range of temperature comprised between 17 °C and 30 °C;
b) isolating the polymorphic form A of the compound of formula (Ia) obtained in previous step; and
c) removing the solvent from the polymorphic form A of the compound of formula (Ia) obtained.

15. A polymorphic form C of a compound according to claim 3, **characterized by** having an X-ray diffractogram that comprises characteristic peaks at approximately 8.8, 9.5, 14.4, 20.3 and 23.0 degrees 2 theta.

16. The polymorphic form according to claim 15, further **characterized by** an X-ray diffractogram as in FIG.3.

17. The polymorphic form according to any of the claims 15-16, further **characterized by** a Differential Scanning Calorimetry (DSC) that comprises a melting peak at 148 °C.

18. A preparation process of the polymorphic form C of the compound of formula (Ia) defined in any of the claims 15-17, **characterized by** comprising the following steps:
a) mixing a compound of formula (Ia) in a solvent system selected from the group consisting of (C₂-C₄)-alkanoates of (C₁-C₄)-alkyl heating up to the boiling point of the solvent system and cooling to a temperature comprised between 0-4 °C;
b) isolating the polymorphic form C of the compound of formula (Ia) obtained in the previous step; and
c) removing the solvent from the polymorphic form C of the compound of formula (Ia) obtained.

19. The preparation process according to claim 18, wherein the step a) is carried out with a solvent system selected from the group consisting of 1-butyl acetate, tert-butyl acetate, ethyl acetate and isopropyl acetate.

20. A pharmaceutical composition **characterized by** comprising a therapeutically effective amount of a compound as defined in any of the claims 1-5, 10-12 and 15-17, together with appropriate amounts of pharmaceutically acceptable excipients or carriers.

21. Use of a compound of formula (I), compound of formula (Ia), and any of the polymorphs A, B or C of compound of fomula (Ia), for the preparation of a medicament for the treatment of bacterial infections of an animal or human.

22. The use according to claim 21, wherein the bacterial infection is caused by Staphylococcus aureus clinical strain cfr methylase.
